# EUROPEAN PATENT SPECIFICATION

(11) **EP 4 328 324 B1**
(45) Date of publication and mention of the grant of the patent: **25.03.2026**
(21) Application number: 23207780.0
(22) Date of filing: 17.11.2016
(51) Int. Cl.: C12Q 1/6883

(54) **METHODS OF DIAGNOSING EPILEPSY**
VERFAHREN ZUR DIAGNOSE VON EPILEPSIE
PROCÉDÉS DE DIAGNOSTIC DE L'ÉPILEPSIE

(30) Priority: 18.11.2015 US 201562257068 P
(43) Date of publication of application: 28.02.2024
(62) Divisional of application: 16867175.8
(73) Proprietor: Dignity Health, San Francisco, CA 94107 (US)
(72) Inventor: TREIMAN, David, Phoenix, Arizona (US); SCHOOLEY, Dustin, Glendale, Arizona (US)
(74) Representative: Hutter, Anton

(56) References cited:
- WO-A2-2014/096418
- US-A1- 2013 022 982
- SCHER ANN I. ET AL: "MTHFR C677T Genotype As a Risk Factor for Epilepsy Including Post-Traumatic Epilepsy in a Representative Military Cohort", JOURNAL OF NEUROTRAUMA., vol. 28, no. 9, 1 September 2011 (2011-09-01), US, pages 1739 - 1745, XP093141525, ISSN: 0897-7151, Retrieved from the Internet <URL:https://dx.doi.org/10.1089/neu.2011.1982> DOI: 10.1089/neu.2011.1982
- PITKÄNEN ASLA ET AL: "Epilepsy Related to Traumatic Brain Injury", NEUROTHERAPEUTICS, SPRINGER INTERNATIONAL PUBLISHING, CHAM, vol. 11, no. 2, 20 February 2014 (2014-02-20), pages 286 - 296, XP035315351, ISSN: 1933-7213, [retrieved on 20140220], DOI: 10.1007/S13311-014-0260-7
- NAGARAJA BALAKATHIRESAN ET AL: "MicroRNA Let-7i Is a Promising Serum Biomarker for Blast-Induced Traumatic Brain Injury", JOURNAL OF NEUROTRAUMA., vol. 29, no. 7, 1 May 2012 (2012-05-01), US, pages 1379 - 1387, XP055356447, ISSN: 0897-7151, DOI: 10.1089/neu.2011.2146
- EL-NAGGAR H ET AL: "PLASMA MICRORNA PROFILING IDENTIFIES POTENTIAL BIOMARKERS OF HUMAN TEMPORAL LOBE EPILEPSY", EPILEPSIA, & 31ST INTERNATIONAL EPILEPSY CONGRESS; ISTANBUL, TURKEY; SEPTEMBER 05 -09, 2015, vol. 56, no. Suppl. 1, Sp. Iss. SI, 1 February 2015 (2015-02-01), pages 116, XP002789423

## Description

### FIELD OF THE INVENTION

The present disclosure is in the medical and health field, specifically diagnosis and therapies related to epilepsy.

### BACKGROUND OF THE DISCLOSURE

Epilepsy is a group of neurological diseases characterized by epileptic seizures. Epilepsy affects millions of people worldwide. Epileptic seizures are episodes that can vary from brief and nearly undetectable to long periods of vigorous shaking. Status epilepticus (SE) is an epileptic seizure where the subject suffers long periods of vigorous shaking - often greater than five minutes without returning to normal between them.

People who suffer Traumatic Brain Injury (TBI) or brain injury - such as through trauma, stroke, or infection - are at an increased risk of developing epilepsy. TBI is a common cause of epilepsy in young adults. Usually 5-50% of patients with TBI develop post traumatic epilepsy (PTE).

Currently there are no available methods to predict which of the 5-50% of patients with TBI will develop epilepsy, and which of the 50-95% of patients will not. Moreover SE continues to be a dangerous condition. Thus there exists a need in the art for a method of diagnosing susceptibility to neurological disorders such as epilepsy.

### SUMMARY OF THE DISCLOSURE

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (or for diagnosis).

Embodiments of the present disclosure include methods of diagnosing susceptibility to epilepsy in an individual, comprising: obtaining a sample from the individual; assaying the sample to determine the presence or absence of one or more biomarkers of epilepsy; and diagnosing susceptibility to epilepsy in the individual based on the presence of one or more biomarkers of epilepsy. In one embodiment, the presence of one or more biomarkers comprises an abnormal expression of micro RNA (miRNA) relative to a healthy subject. In one embodiment, the one or more biomarkers comprises an up-regulation of miRNAs Let-7d-5p, miR-340-3p, miR-484, miR-151, and/or miR-350 relative to a healthy subject. In one embodiment, the one or more biomarkers comprises a down-regulation of miRNA miR-770-5p. In one embodiment, the one or more biomarkers comprises an up-regulation of miRNAs miR-139-3p, miR-2985, and/or miR-101a-5p. In one embodiment, the one or more biomarkers comprises miRNAs miR-206-3p, miR-760-3p, miR-383-5p, miR-294, and/or miR-328a-5p. In one embodiment, the epilepsy is post traumatic epilepsy (PTE). In one embodiment, the individual has previously suffered from traumatic brain injury (TBI). In one embodiment, the one or more biomarkers comprise SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 70% to 80% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 80% to 90% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 90% to 100% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the individual is human. In one embodiment, the individual is a rodent. In one embodiment, the sample is a blood sample. In one embodiment, the individual has received head trauma and/or one or more concussions. In one embodiment, the individual has received a brain insult, stroke, intracranial hemorrhage, tumor, infection, and/or de novo status epilepticus. In one embodiment, assaying the sample comprises (i) contacting the sample with oligonucleotide probes, wherein the oligonucleotide probes specifically hybridize to the polynucleotides of SEQ. ID. NOs. 1-15 in the sample, and wherein the oligonucleotides are labeled with at least one fluorescent dye; (ii) detecting the fluorescent signals from the hybridization complex formed by the oligonucleotide probes and the polynucleotides in the sample; and (iii) detecting the presence of one or more biomarkers of epilepsy based upon the detected fluorescent signals. In one embodiment, the detected fluorescent signals are at least 8-fold higher than a fluorescent signal generated in a negative healthy subject which has an absence of biomarkers of epilepsy.

Embodiments of the present disclosure further include kits for diagnostic use, comprising a single diagnostic panel consisting essentially of one, two, three, four, or five of the following biomarkers: Let-7d-5p, miR-340-3p, miR-484, miR-151, miR-350, miR-770-5p, miR-139-3p, miR-2985, miR-101a-5p, miR-206-3p, miR-760-3p, miR-383-5p, miR-294, and miR-328a-5p. In one embodiment, the single diagnostic panel consists essentially of three or four of the following biomarkers: Let-7d-5p, miR-340-3p, miR-484, miR-151, miR-350, and miR-770-5p. In one embodiment, the single diagnostic panel consists essentially of three or four of the following biomarkers with 70% to 80% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and SEQ ID NO: 15. In one embodiment, the single diagnostic panel consists essentially of three, four, or five of the following biomarkers with 80% to 90% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and SEQ ID NO: 15. In one embodiment, the biomarkers are indicative of epilepsy.

Embodiments of the present disclosure also include methods of managing treatment of a neurological condition in an individual, comprising: obtaining a sample from the subject; assaying the sample to determine the presence or absence of one or more biomarkers selected from the group consisting of: Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p; diagnosing susceptibility to epilepsy based on the presence of the one or more biomarkers; and treating the individual. In one embodiment, treating the individual comprises administering an appropriate epileptogenic pathway inhibitor. In one embodiment, the presence of one or more biomarkers comprises an up-regulation of miRNAs Let-7d-5p, miR-340-3p, miR-484, miR-151, and/or miR-350 relative to a healthy subject. In one embodiment, the presence of one or more biomarkers comprises a down-regulation of miRNA miR-770-5p. In one embodiment, the presence of one or more biomarkers comprises an up-regulation of miRNAs miR-139-3p, miR-2985, and/or miR-101a-5p. In one embodiment, the individual has received a brain insult, stroke, intracranial hemorrhage, tumor, infection, and/or de novo status epilepticus.

Other features and advantages of the invention will become apparent from the following detailed description, taken in conjunction with the accompanying drawings, which illustrate, by way of example, various embodiments of the invention.

### DESCRIPTION OF THE DRAWINGS

Exemplary embodiments are illustrated in referenced figures. It is intended that the embodiments and figures disclosed herein are to be considered illustrative rather than restrictive.

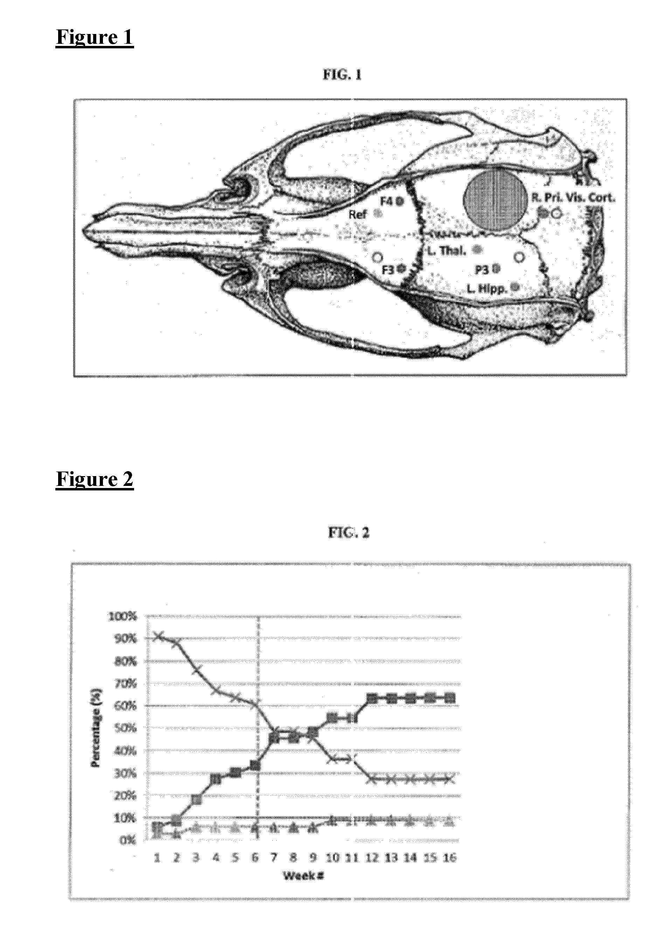

**FIG. 1** illustrates, in accordance with embodiments herein, a view of a rat skull showing the position where the 6 mm piston was made to strike the surface of the brain, and showing the positions where the four cortical electrodes, the two hippocampal electrodes and the two centromedial thalamic nuclei electrodes were implanted.
**FIG. 2** illustrates, in accordance with embodiments herein, seizure development in a Rat CCI model. Seizure development in epileptic rats are illustrated by the symbol "□," single seizure development is illustrated by the symbol "Δ," and no seizures are illustrated by the symbol "×."

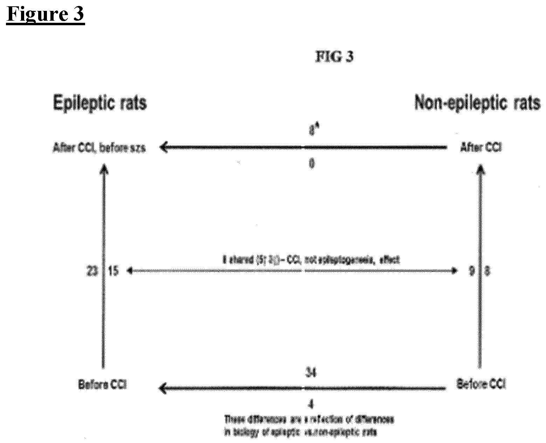

**FIG. 3** illustrates, in accordance with embodiments herein, isolation of the effect of epileptogenesis from the effect of CCI. The numbers on the inside (0, 9, 34, and 15) refer to the number of down-regulated miRNAs, whereas the numbers on the outside (8*, 8, 4, and 23) refer to the number of up-regulated miRNAs. The "8*" in the upper half of the figure illustrates that expression of these eight miRNAs were upregulated compared with non-epileptic post-CCI controls. Expression of five of them changed from baseline in ER but not in NER. All five were upregulated in ER after CCI but before seizure onset compared with NER after CCI. Thus these five miRNAs, up-regulated in ER after CCI but before onset of seizures, are predictive of the development of seizures in these rats.

### DETAILED DESCRIPTION

The invention is as defined by the claims and any other aspects, configurations or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (or for diagnosis).

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 7th ed., J. Wiley & Sons (New York, NY 2013); and Sambrook and Russel, Molecular Cloning: A Laboratory Manual 4th ed., Cold Spring Harbor Laboratory Press (Cold Spring Harbor, NY 2012), provide one skilled in the art with a general guide to many of the terms used in the present application. One skilled in the art will recognize many methods and materials similar or equivalent to those described herein, which could be used in the practice of the present invention. Indeed, the present invention is in no way limited to the methods and materials described.

As used herein, the term "epilepsy" contemplates a chronic disease having recurrent seizures which occur as a result of a sudden excessive electrical discharge in a group of nerve cells. Epilepsy includes Post-Traumatic Epilepsy (PTE). PTE is a form of epilepsy that results from brain damage caused by physical trauma to the brain (traumatic brain injury, abbreviated TBI). Epilepsy also includes status epilepticus (SE), which in one embodiment refers to an epileptic seizure of greater than five minutes or more than one seizure within a five minute period without the person returning to normal between them.

As used herein, the term "epileptogenesis" contemplates the biochemical, genetic, histological or other structural or functional processes or changes that make nervous tissue, including the central nervous system (CNS) susceptible to recurrent, spontaneous seizures. The term "epileptogenesis" also contemplates the changes and processes that contribute to the clinical progression observed in some epilepsies.

As used herein, the terms "microRNA," "miR," or "miRNA" contemplates a small, single stranded, non-coding RNA molecule. In some embodiments, a miRNA comprises 19-25 nucleotides. In some embodiments, the miRNA functions in RNA silencing and post-transcriptional regulation of gene expression. A "miR gene product," "microRNA," "miR," or "miRNA" further refers to the unprocessed or processed RNA transcript from a miR gene. As the miR gene products are not translated into protein, the term "miR gene products" does not include proteins. The unprocessed miR gene transcript is also called a "miR precursor," and typically comprises an RNA transcript of about 70-100 nucleotides in length. The miR precursor that can be processed by digestion with an RNAse (for example, Dicer, Argonaut, RNAse III) into an active miRNA molecule is also referred to as the "processed" miR gene transcript or "mature" miRNA.

As used herein, the term "biomarker" contemplates any substance, structure, or process that can be measured in the subject or its products and influence or predict the incidence of outcome or disease. In one embodiment, the biomarker used herein refers to a biological compound related to the progressive development of PTE.

As used herein, the terms "up-regulate" or "up-regulation" refers to a process by which a cell increases the quantity of a cellular component. Similarly, the terms "down-regulate" or "down-regulation" refers to a process by which a cell decreases the quantity of a cellular component. As an illustrative example, up-regulating a miRNA means that the amount of miRNA in the cell or the function of the miRNA in the cell has been increased.

As used herein, "treatment" and "treating," refers to both therapeutic treatment and prophylactic or preventative measures, wherein the object is to prevent or slow down (lessen) or at least partially ameliorate the targeted pathologic condition or disorder even if the treatment is ultimately unsuccessful. Those in need of treatment include those already with the disorder as well as those prone to have the disorder or those in whom the disorder is to be prevented.

The term "subject" or "patient," as used herein, refers to an animal, preferably a mammal, including a dog, cat, monkey, horse, cow, sheep, or pig, and, in certain preferred embodiments, a human.

The terms "sample" or "biological sample", as used herein, refers to a sample that has been obtained from a patient. The sample may be of any biological tissue, cells or fluid. Such samples include, but are not limited to, sputum, blood, serum, plasma, blood cells (e.g., white cells), tissue, nipple aspirate, core or fine needle biopsy samples, cell-containing body fluids, free floating nucleic acids, urine, peritoneal fluid, and pleural fluid, or cells there from.

The term "control sample," as used herein, refers to any clinically relevant comparative sample, including, for example, a sample from a healthy subject not afflicted with a neurological or other disease, a sample from a subject having a less severe or slower progressing neurological or other disease than the subject to be assessed, a sample from a subject having some other type of neurological or other disease, a sample from a subject prior to treatment, and the like. A control sample may include a sample derived from one or more subjects. A control sample may also be a sample made at an earlier time point from the subject to be assessed. For example, the control sample could be a sample taken from the subject to be assessed before the onset of the neurological disorder, or other disease, at an earlier stage of disease, or before the administration of treatment or of a portion of treatment. A control sample can be a purified sample, a chemical compound, protein, and/ or polynucleotide provided with a kit.

As used herein, the term "pharmaceutical composition" refers to a mixture or solution comprising a therapeutic agent or combination of therapeutic agents to be administered in the selected dosage form to a subject in treating a disease or condition indicated. The term "therapeutic agent" refers to a pharmaceutical agent including any natural or synthetic chemical, biochemical, or biological substance that subsequent to its application has at least one desired pharmaceutical or therapeutic effect.

The pharmaceutical compositions according to the disclosure can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluents, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The terms "polynucleotide" and "oligonucleotide," used interchangeably herein, refer generally to linear polymers of natural or modified nucleosides, including deoxyribonucleosides, ribonucleosides, alpha-anomeric forms thereof, and the like, usually linked by phosphodiester bonds or analogs thereof ranging in size from a few monomeric units, e.g. 2-4, to several hundreds of monomeric units. When a polynucleotide is represented by a sequence of letters, such as "ATGCCTG," it will be understood that the nucleotides are in 5'->3' order from left to right. Polynucleotide as used herein also includes abasic sugarphosphate or sugar- phosphorothioate polymers. In one embodiment, the terms polynucleotide or oligonucleotide refers to microRNA.

As further disclosed herein, the inventors have disclosed a novel method for predicting the development of epilepsy after traumatic brain injury or other epileptogenic brain insults such as stroke, infection, tumor, status epilepticus, cerebral hypoxia, Alzeimer's disease etc. Thus, in one embodiment disclosed herein is a method of diagnosing susceptibility to epilepsy in an individual, comprising: obtaining a sample from the individual; assaying the sample to determine the presence or absence of one or more biomarkers of epilepsy; and diagnosing susceptibility to epilepsy in the individual based on the presence of one or more biomarkers of epilepsy. In one embodiment, the presence of one or more biomarkers comprises an abnormal expression of micro RNA (miRNA) relative to a healthy subject. In one embodiment, the one or more biomarkers comprises an up-regulation of miRNAs Let-7d-5p, miR-340-3p, miR-484, miR-151, and/or miR-350 relative to a healthy subject. In one embodiment, the one or more biomarkers comprises a down-regulation of miRNA miR-770-5p. In one embodiment, the one or more biomarkers comprises an up-regulation of miRNAs miR-139-3p, miR-2985, and/or miR-101a-5p. In one embodiment, the one or more biomarkers comprises miRNAs miR-206-3p, miR-760-3p, miR-383-5p, miR-294, and/or miR-328a-5p. In one embodiment, the epilepsy is post traumatic epilepsy (PTE). In one embodiment, the individual has previously suffered from traumatic brain injury (TBI). In one embodiment, the one or more biomarkers comprise SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 70% to 80% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 80% to 90% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the one or more biomarkers exhibit between 90% to 100% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and/or SEQ ID NO: 15. In one embodiment, the individual is human. In one embodiment, the individual is a rodent. In one embodiment, the sample is a blood sample. In one embodiment, the individual has received head trauma and/or one or more concussions. In one embodiment, the individual has received a brain insult, stroke, intracranial hemorrhage, tumor, infection, and/or de novo status epilepticus. In one embodiment, assaying the sample comprises (i) contacting the sample with oligonucleotide probes, wherein the oligonucleotide probes specifically hybridize to the polynucleotides of SEQ. ID. NOs. 1-15 in the sample, and wherein the oligonucleotides are labeled with at least one fluorescent dye; (ii) detecting the fluorescent signals from the hybridization complex formed by the oligonucleotide probes and the polynucleotides in the sample; and (iii) detecting the presence of one or more biomarkers of epilepsy based upon the detected fluorescent signals. In one embodiment, the detected fluorescent signals is at least 8-fold higher than a fluorescent signal generated in a negative control sample which has an absence of biomarkers of epilepsy.

In another embodiment disclosed herein is a kit for diagnostic use, comprising a single diagnostic panel consisting essentially of one, two, three, four, or five of the following biomarkers: Let-7d-5p, miR-340-3p, miR-484, miR-151, miR-350, miR-770-5p, miR-139-3p, miR-2985, miR-101a-5p, miR-206-3p, miR-760-3p, miR-383-5p, miR-294, and miR-328a-5p. In one embodiment, the single diagnostic panel consists essentially of three or four of the following biomarkers: Let-7d-5p, miR-340-3p, miR-484, miR-151, miR-350, and miR-770-5p. In one embodiment, the single diagnostic panel consists essentially of three or four of the following biomarkers with 70% to 80% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO:9, SEQ ID NO: 10, SEQ ID NO: 11, SEQ ID NO: 12, SEQ ID NO: 13, SEQ ID NO: 14, and SEQ ID NO: 15. In one embodiment, the single diagnostic panel consists essentially of three, four, or five of the following biomarkers with 80% to 90% sequence identity to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and SEQ ID NO: 15. In one embodiment, the biomarkers are indicative of epilepsy.

In another embodiment, disclosed herein is a method of managing treatment of a neurological condition in an individual, comprising obtaining a sample from the subject; assaying the sample to determine the presence or absence of one or more biomarkers selected from the group consisting of: Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p; diagnosing susceptibility to epilepsy based on the presence of the one or more biomarkers; and treating the individual. In one embodiment, treating the individual comprises administering an appropriate epileptogenic pathway inhibitor. In one embodiment, the presence of one or more biomarkers comprises an up-regulation of miRNAs Let-7d-5p, miR-340-3p, miR-484, miR-151, and/or miR-350 relative to a healthy subject. In one embodiment, the presence of one or more biomarkers comprises a down-regulation of miRNA miR-770-5p. In one embodiment, the presence of one or more biomarkers comprises an up-regulation of miRNAs miR-139-3p, miR-2985, and/or miR-101a-5p. In one embodiment, the individual has received a brain insult, stroke, intracranial hemorrhage, tumor, infection, and/or de novo status epilepticus.

Further disclosed herein are methods of diagnosing susceptibility to a neurological disorder in a subject, comprising obtaining a sample from the subject; assaying the sample to determine the presence or absence of one or more polynucleotide biomarkers; and diagnosing susceptibility to the neurological disorder based on the presence of the one or more polynucleotide biomarkers. In one embodiment, the neurological disorder is epilepsy. In one embodiment, the epilepsy is Post-Traumatic Epilepsy (PTE). In one embodiment, the epilepsy is status epilepticus. In one embodiment, the polynucleotide biomarker is a RNA. In one embodiment, the polynucleotide biomarker is a micro-RNA. In one embodiment, the polynucleotide biomarkers comprise a RNA selected from the group consisting of: Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p, or a polynucleotide that exhibits at least 50%, preferably at least 60%, more preferably at least 70%, more preferably at least 80%, more preferably at least 90%, and most preferably at least 95%sequence identity to one of the sequences. In one embodiment, the method further comprises up-regulation of miRNA Let-7d-5p, miR-340-3p, miR-484, miR-151, and/or miR-350. In one embodiment, the method further comprises up-regulation of miRNA miR-139-3p, miR-2985, or miR-101a-5p. In one embodiment, the method further comprises down-regulation of miRNA miR-770-5p. In one embodiment, the method further comprises detecting the up-regulation or down-regulation of one or more miRNAs. In one embodiment, the one or more polynucleotide biomarkers comprise SEQ ID NOs. 1-15. In one embodiment, the one or more polynucleotide biomarkers comprise a polynucleotide that exhibits at least 50%, preferably at least 60%, more preferably at least 70%, more preferably at least 80%, more preferably at least 90%, and most preferably at least 95% sequence identity to one of the sequences described as SEQ ID NOs 1-15 herein. In one embodiment, the subject is human. In one embodiment, the sample is a blood sample. In one embodiment, the subject has received head trauma and/or one or more concussions. In one embodiment, the subject has received a brain insult. In one embodiment, the brain insult is stroke, intracranial hemorrhage, tumor, infection, and/or de novo status epilepticus.

Also disclosed herein are methods for diagnosing a neurological disorder in a subject, comprising: (a) identifying the presence or absence of an up-regulation of a polynucleotide in a blood sample taken from a subject relative to a healthy individual; and (b) diagnosing the neurological disorder based on the presence of the polynucleotide. In one embodiment, the neurological disorder is PTE. In one embodiment, the neurological disorder is epilepsy. In one embodiment, the subject has received a brain insult. In one embodiment, the brain insult is stroke, intracranial hemorrhage, tumor, infection, and/or de novo status epilepticus.

In one embodiment, disclosed herein is a composition, comprising: one or more polynucleotides according to SEQ ID NOs 1-15; and an acceptable carrier. In another embodiment, disclosed herein is a method of managing treatment of traumatic brain injury (TBI), comprising: diagnosing susceptibility to post traumatic epilepsy (PTE) in the individual, monitoring those individuals for symptoms related to epilepsy, and administering the appropriate epileptogenic pathway inhibitor.

In one embodiment, disclosed herein is a method of treating a neurological condition in a subject, comprising: determining the presence of one or more polynucleotide biomarkers indicative of the neurological condition; and treating the subject. In another embodiment, disclosed herein is a method of reducing the incidence, frequency, duration, and/or severity of a neurological disorder, comprising: diagnosing susceptibility to the neurological disorder in a subject comprising obtaining a sample from the subject, assaying the sample to determine the presence or absence of one or more polynucleotide biomarkers, and diagnosing susceptibility to the neurological disorder based on the presence of the one or more polynucleotide biomarkers; and treating the subject with a therapeutically effective dosage of a pharmaceutical composition. In another embodiment, disclosed herein is a method of prognosing a neurological condition in a subject, comprising: obtaining a sample from the subject; assaying the sample to determine the presence or absence of one or more polynucleotide biomarkers associated with the neurological condition; and prognosing a severe form of the neurological condition based on the presence of the one or more polynucleotide biomarkers associated with epilepsy. In one embodiment, treating the subject comprises administering an effective anti-epileptogenic agent. In one embodiment, treating the subject comprises inhibiting one or more abnormal pathways related to the presence of the one or more polynucleotide biomarkers. In one embodiment, the neurological disorder is epilepsy. In one embodiment, the epilepsy is Post-Traumatic Epilepsy (PTE). In one embodiment, the epilepsy is status epilepticus. In one embodiment, the polynucleotide biomarker is a RNA. In one embodiment, the polynucleotide biomarker is a micro-RNA. In one embodiment, the polynucleotide biomarkers comprise a RNA selected from the group consisting of: Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p, or a polynucleotide that exhibits at least 50%, preferably at least 60%, more preferably at least 70%, more preferably at least 80%, more preferably at least 90%, and most preferably at least 95% sequence identity to one of the sequences. In one embodiment, the method further comprises up-regulation of Let-7d-5p, miR-340-3p, miR-484, miR-151, and/or miR-350. In one embodiment, the method further comprises up-regulation of miR-139-3p, miR-2985, or miR-101a-5p. In one embodiment, the method further comprises down-regulation of miR-770-5p. In one embodiment, the one or more polynucleotide biomarkers comprise SEQ. ID. NOs. 1-15. In one embodiment, the one or more polynucleotide biomarkers comprise a polynucleotide that exhibits at least 50%, preferably at least 60%, more preferably at least 70%, more preferably at least 80%, more preferably at least 90%, and most preferably at least 95% sequence identity to one of the sequences described as SEQ ID NOs 1-15 herein. In one embodiment, the subject is human. In one embodiment, the sample is a blood sample. In one embodiment, the subject has received head trauma and/or one or more concussions. In one embodiment, the subject has received a brain insult. In one embodiment, the brain insult is stroke, intracranial hemorrhage, tumor, infection, and/or de novo status epilepticus.

Various examples exist for miRNA biomarkers, as for example, Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p. As readily apparent to one of skill in the art, various polynucleotide sequences may also be used and the invention is in no way limited to only the specific versions of miRNA biomarkers listed herein. Similarly, as readily understood by one of skill in the art, various examples of miRNA sequences with similar homology may be found in different species, and the miRNA biomarkers are in no way limited to only the subject species described herein. Some additional, non-limiting examples of miRNA sequences provided herein include the following SEQ. ID. NOS: 1-15 as shown in Table 1.

**Table 1. Illustrative list of RNA sequences**

| **Sequence #** | **Sequence name** | **Sequence** |
|---|---|---|
| SEQ ID NO. 1 | let-7d-3p | cuauacgacc ugcugccuuu cu |
| SEQ ID NO. 2 | miR-340-3p | uccgucucag uuacuuuaua gc |
| SEQ ID NO. 3 | miR-350-3p | uucacaaagc ccauacacuu uc |
| SEQ ID NO. 4 | miR-350-5p | aaagugcaug cgcuuuggg |
| SEQ ID NO. 5 | miR-484 | ucaggcucag uccccucccg au |
| SEQ ID NO. 6 | miR-151a-3p | cuagacugaa gcuccuugag g |
| SEQ ID NO. 7 | miR-139-3p | uggagacgcg gcccuguugg agu |
| SEQ ID NO. 8 | miR-206-3p | uggaauguaa ggaagugugu gg |
| SEQ ID NO. 9 | miR-760-3p | cggcucuggg ucugugggga |
| SEQ ID NO. 10 | miR-2985 | uguuauagua ucccaccuac cc |
| SEQ ID NO. 11 | miR-383-5p | agaucagaag gugauugugg cu |
| SEQ ID NO. 12 | miR-101-5p | caguuaucac agugcugaug cu |
| SEQ ID NO. 13 | miR-770-5p | uccaguacca cgugucaggg cca |
| SEQ ID NO. 14 | miR-294 | cucaaaaugg aggcccuauc u |
| SEQ ID NO. 15 | miR-328a-5p | gggggggcag gaggggcuca ggg |

Similarly, as understood by one of skill in the art, additional biomarkers may be used, such as for example, other compounds in the same pathway as the various miRNA biomarkers described herein, and the invention is in no way limited to only miRNA polynucleotides or molecules.

In various embodiments, the disclosure may include additional forms of brain injury/insult before diagnosis, treatment and/or prognosis of epilepsy, and the disclosure is in no way limited to only Post-Traumatic Epilepsy (PTE). For example, brain insult may include stroke, intracranial hemorrhage, tumor, infection, and/or de novo status epilepticus. In one embodiment, for example, the subject may suffer a stroke, wherein one or more biomarkers indicative of epilepsy may be used to determine a future onset of epilepsy and/or diagnose epilepsy.

In some embodiments, the present invention describes the use of miRNAs as biomarkers to predict the development of PTE. In some of these embodiments, the miRNA is a specific whole blood miRNA.

In one embodiment, described herein is a method of diagnosing susceptibility to PTE in a mammal, comprising (a) identifying the presence or absence of an up-regulation or down-regulation of a micro-RNA (miRNA) in a blood sample of the mammal relative to a control sample; and (b) diagnosing that the mammal is susceptible to PTE if up-regulation of miRNA is present. In some of these embodiments, the mammal is human. In some embodiments, the miRNA that is up-regulated or down-regulated is Let-7d-5p, miR-340-3p, miR-484, miR-151, miR-350, miR-139-3p, miR-2985, miR-101a-5p, miR-770-5p, miR-206-3p, miR-760-3p, miR-383-5p, miR-294, and miR-328a-5p. In some embodiments, the miRNA comprises a nucleic acid sequence which exhibits at least 50% identity to one of the sequences according to SEQ ID NOs 1-15.

In some embodiments, provided herein is a method for diagnosing Post-Traumatic Epilepsy in a mammal, comprising (a) identifying the presence or absence of an up-regulation or down-regulation of a micro-RNA (miRNA) in a kit
of the mammal relative to a second mammal without PTE; and (b) diagnosing that the first mammal is susceptible to PTE if up-regulation or down-regulation of miRNA is present. In some embodiments, provided herein is a method of treating Post-Traumatic Epilepsy in a patient comprising: (i) identifying the presence or absence of an up-regulation or down-regulation of a micro-RNA (miRNA) in a blood sample of the patient relative to an individual without PTE; (ii) diagnosing that the patient is susceptible to PTE if up-regulation or down-regulation of miRNA is present, and (iii) treating the patient with a therapeutically effective dosage of a pharmaceutical composition useful for the treatment of epilepsy. In various embodiments, provided herein is a composition comprising one or more miRNA according to SEQ ID NOs 1-15, and a pharmaceutically acceptable carrier. In some embodiments, provided herein is a method of managing individuals with TBI by putting them on an epileptogenic pathway inhibitor comprising: (a) diagnosing susceptibility to PTE in the individual as described herein, and (b) monitoring those individuals for symptoms related to epilepsy. In some embodiments, contemplated herein is a method of reducing the incidence, frequency, duration, or severity of PTE comprising diagnosing susceptibility to PTE in an individual as described herein, and treating the individual with a therapeutically effective amount of a pharmaceutical composition useful for the treatment of epilepsy.

The present disclosure is also directed to a kit to diagnose and treat epilepsy. The kit is useful for practicing the inventive method of diagnosing susceptibility to epilepsy, or prognosing traumatic brain injury, for example. The kit is an assemblage of materials or components, including at least one of the inventive compositions. Thus, in some embodiments the kit contains a composition including biomarkers of various miRNA molecules or compounds for the detection of various miRNA biomarkers associated with epilepsy, as described above.

The exact nature of the components configured in the inventive kit depends on its intended purpose. For example, some embodiments are configured for the purpose of treating epilepsy or brain injury. In one embodiment, the kit is configured particularly for the purpose of treating mammalian subjects. In another embodiment, the kit is configured particularly for the purpose of treating human subjects. In further embodiments, the kit is configured for veterinary applications, treating subjects such as, but not limited to, farm animals, domestic animals, and laboratory animals.

Instructions for use may be included in the kit. "Instructions for use" typically include a tangible expression describing the technique to be employed in using the components of the kit to effect a desired outcome, such as to detect the presence of biomarkers. Optionally, the kit also contains other useful components, such as, diluents, buffers, pharmaceutically acceptable carriers, syringes, catheters, applicators, pipetting or measuring tools, bandaging materials or other useful paraphernalia as will be readily recognized by those of skill in the art.

The materials or components assembled in the kit can be provided to the practitioner stored in any convenient and suitable ways that preserve their operability and utility. For example, the components can be in dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as inventive compositions and the like. The packaging material is constructed by well-known methods, preferably to provide a sterile, contaminant-free environment. As used herein, the term "package" refers to a suitable solid matrix or material such as glass, plastic, paper, foil, and the like, capable of holding the individual kit components. Thus, for example, a package can be a glass vial used to contain suitable quantities of an inventive composition containing one or more therapeutic compositions, or biomarkers. The packaging material generally has an external label which indicates the contents and/or purpose of the kit and/or its components.

Embodiments of the present disclosure are further described in the following examples. The examples are merely illustrative and do not in any way limit the scope of the invention as claimed.

### EXAMPLES

### EXAMPLE 1

### Background

A Traumatic Brain Injury (TBI) is an injury that disrupts the normal function of the brain. It can be caused by a bump, blow, or jolt to the head or a penetrating head injury. Explosive blasts can also cause TBI, particularly among those who serve in the military. In 2010, the Centers for Disease Control and Prevention (CDC) estimated that TBIs accounted for approximately 2.5 million emergency department (ED) visits, hospitalizations, and deaths in the United States, either as an isolated injury or in combination with other injuries.

People who suffer TBI or brain injury - such as through trauma, stroke, or infection - are at an increased risk of developing epilepsy. TBI is the most common cause of epilepsy in young adults. TBI is a major problem for soldiers who serve in the military and TBI may ultimately lead to Post Traumatic Epilepsy (PTE). More than 30,000 new cases of post-traumatic epilepsy are reported per year in the U.S. alone, and at least 600,000 new cases are reported world-wide. Increasing evidence suggests even mild TBI, including repeated concussions, substantially increases the risk for PTE. TBI accounts for more than 5% of all epilepsy and more than 20% of symptomatic epilepsy. Usually 5-50% of patients with TBI develop PTE.

However, despite such progress, currently there are no available methods to predict which of the 5-50% of patients with TBI will develop epilepsy, and which of the 50-95% of patients will not, so that only patients at risk are given early treatment with anti-epileptic or anti-epileptogenic drugs. In one embodiment, the inventors herein have disclosed several markers that predict a risk for developing epilepsy among patients. In one embodiment, the epilepsy is caused due to brain injury or other brain insults. In one embodiment, the epilepsy is status epilepticus.

### EXAMPLE 2

### Generally

In one embodiment, the inventors have developed a new method or technique that predicts the development of epilepsy after traumatic brain injury by detecting a change in expression in one or more miRNAs. In one embodiment, the change in expression corresponds to an up-regulation or down regulation of a group of microRNAs. Patients with TBI or other epileptogenic brain insults such as stroke, infection, tumor, status epilepticus, cerebral hypoxia, Alzheimer's disease, etc., could be followed by obtaining serial blood samples to predict which of these patients are likely to develop post-traumatic epilepsy (PTE). When effective anti-epileptogenic agents become available only those patients likely to develop epilepsy after brain insult could be treated with such agents to prevent their developing epilepsy and those unlikely to develop epilepsy could avoid such medication.

After TBI only 5-50% of such individuals go on to develop epilepsy. Thus 50-95% of TBI victims do not develop epilepsy. Identifying people at risk for PTE would allow only such people to be subjected to treatment with anti-epileptogenic agents. For other brain insults, the risk is considerably lower (e.g. 5% for Alzheimer's, 15% for cerebral infraction, 30% for cerebral hemorrhage, etc.) so being able to predict epileptogenesis in such individuals would be even more important.

Although there is considerable interest in identifying biomarkers predictive of PTE and development of epilepsy after other brain insults, there are as yet no currently available reliable predictors of post-brain insult epileptogenesis with reasonable specificity or sensitivity.

One advantage of the present disclosure is the use of blood samples in which to detect changes in microRNA expression as opposed to CSF or brain tissue, where it is much harder to obtain serial samples.

### EXAMPLE 3

### Surgical and EEG methods

36 adult male Sprague-Dawley rats, weighing approximately 300 grams each, underwent a 6 mm diameter right parietal craniotomy. As illustrated in Fig. 1, using a pneumatically controlled cortical impact device, a 6 mm diameter piston was made to strike the surface of the brain at a velocity of 5.5 meters per sec. and to penetrate 3 mm into the cortical surface of the brain for a total duration of 55 msec. Four cortical electrodes, two hippocampal electrodes, and two centromedial thalamic nuclei electrodes were implanted, in locations shown in Fig. 1. Starting 10 days after surgery, rats were monitored continuously for 16 weeks using an Xltek EEG recording machine to determine the time of onset, frequency, and duration of spontaneous seizures, as detected by visual inspection of the EEG.

Controlled cortical impact (CCI) model of TBI: 21 out of 33 rats became epileptic; 3 out of the 33 rats had one seizure; and 9 out of the 33 rats did not develop epilepsy. The mean time to develop seizures was 6.14 weeks, with standard deviation of 3.49 weeks; the median was 6.0 weeks and the range was 1-12 weeks. These results are illustrated in Fig. 2 and 3.

miRNA analysis: Blood samples from the mice were collected prior to surgery and 4, 8, 12, and 16 weeks post-surgery and stored on QIAGEN^{®} RNA-protect Animal Blood Tubes. miRNA was isolated from blood samples from the six most epileptic rats (ER) and compared with those from 6 rats that did not develop epilepsy (NER). Blood samples from the draw immediately preceding the first spontaneous seizure were used for the epileptic rats; samples drawn at the same time were used for 6 non-epileptic rats. Thus there were 4 groups for comparison: ER baseline, NER baseline, ER experimental (after CCI, before seizures), NER experimental (after CCI, no seizures, blood sample paired with ER experimental). miRNA was amplified by polymerase chain reaction and analyzed using the ΔΔC_{T} method. Statistical significance: p<0.05.

Table 2 illustrates the number of miRNAs significantly up or down regulated per comparison. Table 2 is illustrated as a figure in Fig. 3

**Table 2: Number of miRNAs significantly up or down regulated per comparison**

| Comparison | Number of miRNAs up regulated | Number of miRNAs down regulated |
|---|---|---|
| Epileptic baseline vs non-epileptic baseline | 34 | 4 |
| Non-epileptic experimental vs. non-epileptic baseline | 9 | 8 |
| Epileptic experimental vs epileptic baseline | 23 | 15 |
| Epileptic experimental vs non-epileptic experimental | 8 | 0 |

Results for the miRNA biomarkers: The inventors isolated the effect of epileptogenesis from the effect of CCI. It was found that the following miRNAs were up-regulated vs. both ER Baseline & NER experimental Groups
1. Let-7d-3p (+1.22 fold change)
2. miR-340-3p (+1.38 fold change)
3. miR-350 (+1.70 fold change)
4. miR-484 (+1.71 fold change)

In some embodiments, Let-7d-3p miRNA was found to regulate Galectin-3, which is up-regulated in microglial cells after pilocarpine-induced SE. In some embodiments, miR-340-3p miRNA was found to regulate the TGFβ pathway implicated in epileptogenesis.

The following miRNA was down-regulated in the epileptic rats baseline vs. epileptic rats experimental group, and up-regulated in epileptic rats experimental group vs. the rats that did not develop epilepsy experimental group.

### 5. miR-151-3p (-1.29 fold change)

These results illustrate that the five miRNAs identified above are biomarkers of epilepsy or epileptogenesis.

### EXAMPLE 4

### Status Epilepticus

Status epilepticus (SE) is a dynamic condition in which EEG patterns and convulsive behavior evolve if seizure activity continues. To better understand the pathophysiology of SE the inventors studied the expression of miRNAs in rats at the five defined EEG stages of SE (as illustrated in Treiman et al., Epilepsy Res 5:49-60, 1990).

To perform miRNA analysis, fresh hippocampal tissue was collected after induction of SE Stage 1, 3, and 5 along with Control hippocampal samples and flash frozen on liquid nitrogen and stored at -80°C. MicroRNA was isolated from the hippocampal tissue of rats from specific EEG stages (1, 3, 5, and control) of lithium-pilocarpine induced SE. Thus there were 4 groups for comparison: ER Stage 1, ER Stage 3, ER Stage 5, and Control. 653 miRNAs were amplified by PCR using QIAGEN^{®} Rat miRNome miScript miRNA PCR arrays and analyzed using the ΔΔC_{T} method. Statistical significance was set at p<0.05. Experimental groups were compared against the Control group and also one another.

The results for the 9 micro RNAs - miR-139-3p, miR-206-3p, miR-760-3p, miR-2985, miR-383-5p, miR-101a-5p, miR-770-5p, miR-294, miR-328a-5p - are shown below.
1. miR-139-3p: C-Stage 1= -1.05 fold change, Stage 1-Stage 3= -1.39 fold change, Stage 3-Stage 5= -1.17 not statistically significant.
2. miR-206-3p: C-Stage 1= -1.84 fold change, Stage 1-Stage 3= -1.02 fold change, Stage 3-Stage 5= -1.03 not statistically significant.
3. miR-760-3p: C-Stage 1= +1.12 fold change, Stage 1-Stage 3= -1.36 fold change, Stage 3-Stage 5= -1.31 NS (C-Stage 5= -1.59 fold change).
4. miR-2985: C-Stage 1= -1.12 fold change, Stage 1-Stage 3= -1.29 fold change, Stage 3-Stage 5= +1.04 fold change. All stages show significant differences.
5. miR-383-5p: C-Stage 1= +1.49 fold change, Stage 1-Stage 3= -1.84 fold change, Stage 3-Stage 5= +1.01 not statistically significant.
6. miR-101a-5p: C-Stage 1= -1.02 fold change, Stage 1-Stage 3= -1.56 not statistically significant, Stage 3-Stage 5= +2.12 fold change (C-Stage 5= +1.34 fold change).
7. miR-770-5p: C-Stage 1 = + 1.18 fold change, Stage 1-Stage 3= -1.50 not statistically significant, Stage 3-Stage 5= +1.90 fold change (C-Stage 5= +1.49 fold change).
8. miR-294: C-Stage 1= -1.31 not statistically significant, Stage 1-Stage 3= -3.62 fold change, Stage 3-Stage 5= +3.78 fold change.
9. miR-328a-5p: C-Stage 1= -2.20 fold change, Stage 1-Stage 3= -1.71 not statistically significant, Stage 3-Stage 5= +3.68 fold change (C-Stage 5= -1.02 fold change).

DOD Comparison results of these micro RNAs are shown below.

miR-139-3p: Epileptic experimental animals were +1.97 fold upregulated vs epileptic baseline. Induced-SE animals from this study show a consistent down-regulation between the various stages.

miR-206-3p: This miRNA as found to be down-regulated versus a healthy specimen in an acute model of status epilepticus. This miRNA did not undergo significant changes in a chronic post-traumatic epilepsy model.

miR-760-3p: This miRNA was found to be generally down-regulated in an acute status epilepticus model except for EEG stage 1 where it was up-regulated when compared to a healthy cohort.

miR-2985: Epileptic experimental animals were +3.51 fold upregulated compared to non-epileptic experimental animals. Induced-SE animals from this study showed down-regulation from C-Stage 1 and Stage 1-Stage 3 but up-regulation from Stage 3-Stage 5. (NS down-regulation from C-Stage 5).

miR-383-5p: When compared to a healthy cohort and between EEG stages of status epilepticus, this miRNA was found to be up-regulated except for EEG stage 3 which was found to return to a near baseline level. Studies have shown that in chronic epilepsy this miRNA is down-regulated compared to healthy individuals.

miR-101a-5p: Epileptic experimental animals exhibited +1.25 fold up-regulation compared to epileptic baseline samples. Induced-SE animals in this study showed down-regulation from C-Stage 1 (significant) and from Stage 1-Stage 3 (NS) and up-regulation from Stage 3-Stage 5 (significant).

miR-770-5p: Non-epileptic experimental animals had a -5.62 fold down-regulation compared to non-epileptic experimental animals. Induced-SE animals from this study showed significant up-regulation from C-Stage 1 (+1.18), non-significant down-regulation from Stage 1-Stage 3 (-1.50), and significant up-regulation from Stage 3-Stage 5 (+1.90). (C-Stage 5 also was significantly up-regulated +1.49). Based on the inventors experimental results in regards to post-traumatic epileptogenesis, mir-770-5p was significantly down-regulated in specimens that did not develop epilepsy when compared to their baseline samples. Individuals that did develop epilepsy did not show the same type of regulation of this particular miRNA and it is likely that down-regulation of mir-770-5p may be a neuroprotective mechanism.

miR-294: This miRNA was found to be down-regulated except when comparing EEG stage 5 samples to EEG stage 3 samples. In this comparison the Stage 5 samples were found to be significantly up-regulated.

miR-328a-5p: When compared to a healthy cohort, this miRNA was found to be down-regulated. However, when comparing EEG Stage 5 to EEG stage 3, Stage 5 was significantly up-regulated.

Based on the results above, in one embodiment, the inventors found that this study identified 9 miRNAs that are markers for epilepsy. These miRNAs (miR-139-3p, miR-2985, miR-101a-5p, miR-770-5p, miR-206-3p, miR-760-3p, miR-383-5p, miR-294, miR-328a-5p) shed light on the pathophysiology of SE along with epilepsy. Moreover these miRNAs are useful in diagnosing the susceptibility of a neurological disorder in a mammal, as well as treating the neurological disorder.

In addition to the above results, in one embodiment, Mir-206 in humans is likely to target brain-derived neurotrophic factor. Moreover, Mir-760-3p may target KCNJ3 which is a potassium voltage-gated channel member. Mir-383-5p may target transforming growth factor-β receptor 1 (TGFBR1) which has been associated with epileptic seizures. Mir-294 may target proteolipid protein 1 (PLP1) which is one of the main proteins found in myelin in the central nervous system. And finally, Mir-328 in humans is likely to target c-type lectin domain family 2 member B (CLEC2B). Members of this family have different functions including inflammatory and immune responses.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

## Claims

1. A method of diagnosing susceptibility to epilepsy in an individual, comprising:
assaying a sample that has been obtained from an individual to determine the presence or absence of at least one mRNA biomarker selected from miR-340-3p, miR-484, and/or miR-350; and
diagnosing susceptibility to epilepsy in the individual based on the presence of miR-340-3p, miR-484, and/or miR-350;
wherein the individual has suffered a traumatic brain injury and assaying the sample comprises (i) contacting the sample with an oligonucleotide probe, wherein the oligonucleotide probe specifically hybridizes to the miRNA biomarker selected from miR-340-3p, miR-484, and/or miR-350 in the sample, and wherein the oligonucleotide is labelled with a fluorescent dye; (ii) detecting the fluorescent signal from the hybridization complex formed by the oligonucleotide probe and the polynucleotide in the sample; and (iii) detecting the presence of the biomarker of epilepsy based upon the detected fluorescent signal, wherein the sample is a blood sample and wherein an up-regulation of the miRNA biomarker selected from miR-340-3p, miR-484, and/or miR-350 relative to a healthy subject is indicative of a susceptibility to epilepsy, and wherein the epilepsy is post traumatic epilepsy (PTE).

2. The method of claim 1, wherein miRNA miR-340-3p exhibits:
i) between 70% to 80% sequence identity to SEQ ID NO: 2,
ii) between 80% to 90% sequence identity to SEQ ID NO: 2; or
iii) between 90% to 100% sequence identity to SEQ ID NO: 2.

3. The method of claim 1, wherein miRNA miR-484 exhibits
i) between 70% to 80% sequence identity to SEQ ID NO: 5,
ii) between 80% to 90% sequence identity to SEQ ID NO: 5; or
iii) between 90% to 100% sequence identity to SEQ ID NO: 5.

4. The method of claim 1, wherein miRNA miR-350 exhibits
i) between 70% to 80% sequence identity to SEQ ID NO: 3 or 4,
ii) between 80% to 90% sequence identity to SEQ ID NO: 3 or 4; or
iii) between 90% to 100% sequence identity to SEQ ID NO: 3 or 4.

5. The method of any one of claims 1-4, further comprising detection of a biomarker comprising SEQ ID NO: 6 (miR-151a-3p).

6. The method of any one of claims 1-3, wherein the individual is human or a rodent.

7. The method of any one of claims 1-3, wherein the individual has received head trauma and/or one or more concussions.

8. Use of a kit for use in diagnosing susceptibility to post traumatic epilepsy (PTE) in a blood sample, the kit comprising compounds for the detection of at least one mRNA biomarker selected from miR-340-3p (SEQ ID NO: 2), miR-484 (SEQ ID NO: 5) and/or miR-350 (SEQ ID No: 3 or 4), or a sequence with 80% to 90% sequence identity to any one of SEQ ID NOs: 2-5, and a control sample from a healthy subject, wherein the sample is a blood sample and wherein detection of up-regulation of miRNA miR-340-3p, miR-484, and/or miR-350 relative to a healthy subject is indicative of a susceptibility to PTE.

9. The use according to claim 8, wherein the kit comprises compounds for the detection of biomarkers consisting essentially of two, three, or all four of the following biomarkers with 80% to 90% sequence identity to SEQ ID NO: 2, SEQ ID NO: 3, SEQ ID NO: 4, SEQ ID NO: 5.

10. The use according to claim 8 or claim 9, wherein the kit further comprises compounds for the detection of miR-151a-3p (SEQ ID NO: 6), or a sequence with 70% to 80% sequence identity to SEQ ID NO: 6.

11. The use according to claim 10, wherein the kit consists essentially of compounds for the detection of two, three or four of the following biomarkers: miR-340-3p, miR-484, miR-350, and/or miR-151-3p.

## Patentansprüche

1. Verfahren zum Diagnostizieren von Anfälligkeit für Epilepsie bei einem Individuum, umfassend:
Untersuchen einer Probe, die von einem Individuum gewonnen wurde, um die Anwesenheit oder Abwesenheit von mindestens einem mRNA-Biomarker, ausgewählt aus miR-340-3p, miR-484 und/oder miR-350, zu bestimmen; und
Diagnostizieren von Anfälligkeit für Epilepsie bei dem Individuum basierend auf dem Vorhandensein von miR-340-3p, miR-484 und/oder miR-350;
wobei das Individuum eine traumatische Hirnverletzung erlitten hat und wobei das Untersuchen der Probe umfasst (i) Inkontaktbringen der Probe mit einer Oligonukleotidsonde, wobei die Oligonukleotidsonde spezifisch mit dem miRNA-Biomarker hybridisiert, der aus miR-340-3p, miR-484 und/oder miR-350 in der Probe ausgewählt ist, und wobei das Oligonukleotid mit einem Fluoreszenzfarbstoff markiert ist; (ii) Nachweisen des Fluoreszenzsignals von dem Hybridisationskomplex, der durch die Oligonukleotidsonde und das Polynukleotid in der Probe gebildet wird; und (iii) Nachweisen des Vorhandenseins des Biomarkers von Epilepsie auf Grundlage des detektierten Fluoreszenzsignals, wobei die Probe eine Blutprobe ist und wobei eine Hochregulation des miRNA-Biomarkers, der aus miR-340-3p, miR-484 und/oder miR-350 ausgewählt ist, relativ zu einem gesunden Subjekt ein Hinweis auf eine Anfälligkeit für Epilepsie ist, und wobei die Epilepsie eine posttraumatische Epilepsie (PTE) ist.

2. Verfahren nach Anspruch 1, wobei miRNA miR-340-3p Folgendes vorweist:
i) zwischen 70 % und 80 % Sequenzidentität mit SEQ ID NO: 2,
ii) zwischen 80 % und 90 % Sequenzidentität mit SEQ ID NO: 2; oder
iii) zwischen 90 % und 100 % Sequenzidentität mit SEQ ID NO: 2.

3. Verfahren nach Anspruch 1, wobei miRNA miR-484 Folgendes vorweist
i) zwischen 70 % und 80 % Sequenzidentität mit SEQ ID NO: 5,
ii) zwischen 80 % und 90 % Sequenzidentität mit SEQ ID NO: 5; oder
iii) zwischen 90 % und 100 % Sequenzidentität mit SEQ ID NO: 5.

4. Verfahren nach Anspruch 1, wobei miRNA miR-350 Folgendes vorweist
i) zwischen 70 % und 80 % Sequenzidentität mit SEQ ID NO: 3 oder 4,
ii) zwischen 80 % und 90 % Sequenzidentität mit SEQ ID NO: 3 oder 4; oder
iii) zwischen 90 % und 100 % Sequenzidentität mit SEQ ID NO: 3 oder 4.

5. Verfahren nach einem der Ansprüche 1 bis 4, ferner umfassend den Nachweis eines Biomarkers,
umfassend SEQ ID NO: 6 (miR-151a-3p).

6. Verfahren nach einem der Ansprüche 1 bis 3, wobei das Individuum ein Mensch oder ein Nagetier ist.

7. Verfahren nach einem der Ansprüche 1 bis 3, wobei das Individuum ein Kopftrauma und/oder eine oder mehrere Gehirnerschütterungen erlitten hat.

8. Verwenden eines Kits zur Verwendung für das Diagnostizieren von Anfälligkeit für posttraumatische Epilepsie (PTE) in einer Blutprobe, wobei das Kit Verbindungen zum Nachweis mindestens eines mRNA-Biomarkers umfasst, ausgewählt aus miR-340-3p (SEQ ID NO: 2), miR-484 (SEQ ID NO: 5) und/oder miR-350 (SEQ ID NR: 3 oder 4) oder einer Sequenz mit 80 % bis 90 % Sequenzidentität mit einer von SEQ ID NOs: 2-5, und einer Kontrollprobe von einem gesunden Subjekt, wobei die Probe eine Blutprobe ist und wobei der Nachweis einer Hochregulation von miRNA miR-340-3p, miR-484 und/oder miR-350 relativ zu einem gesunden Subjekt ein Hinweis auf eine Anfälligkeit für PTE ist.

9. Verwendung nach Anspruch 8, wobei das Kit Verbindungen zum Nachweis von Biomarkern umfasst, die im Wesentlichen aus zwei, drei oder allen vier der folgenden Biomarker mit einer Sequenzidentität von 80 % bis 90 % mit SEQ ID NO: 2, SEQ ID NO: 3, SEQ ID NO: 4, SEQ ID NO: 5 besteht.

10. Verwendung nach Anspruch 8 oder Anspruch 9, wobei das Kit ferner Verbindungen zum Nachweis von miR-151a-3p (SEQ ID NO: 6), oder eine Sequenz mit 70 % bis 80 % Sequenzidentität mit SEQ ID NO: 6 umfasst.

11. Verwendung nach Anspruch 10, wobei das Kit im Wesentlichen aus Verbindungen zum Nachweis von zwei, drei oder vier der folgenden Biomarker besteht: miR-340-3p, miR-484, miR-350 und/oder miR-151-3p.

## Revendications

1. Procédé de diagnostic de la prédisposition à l'épilepsie chez un individu, comprenant :
l'analyse d'un échantillon qui a été prélevé chez un individu pour déterminer la présence ou l'absence d'au moins un biomarqueur ARNm choisi parmi miR-340-3p, miR-484 et/ou miR-350 ; et
le diagnostic de la prédisposition à l'épilepsie chez l'individu sur la base de la présence de miR-340-3p, miR-484 et/ou miR-350 ;
dans lequel l'individu a subi une lésion cérébrale traumatique et l'analyse de l'échantillon comprend (i) la mise en contact de l'échantillon avec une sonde oligonucléotidique, ladite sonde oligonucléotidique s'hybridant spécifiquement au biomarqueur miARN choisi parmi miR-340-3p, miR-484 et/ou miR-350 dans l'échantillon, et ledit oligonucléotide étant marqué avec un colorant fluorescent ; (ii) la détection du signal fluorescent provenant du complexe d'hybridation formé par la sonde oligonucléotidique et le polynucléotide dans l'échantillon ; et (iii) la détection de la présence du biomarqueur de l'épilepsie sur la base du signal fluorescent détecté, ledit échantillon étant un échantillon de sang et une régulation à la hausse du biomarqueur miARN choisi parmi miR-340-3p, miR-484 et/ou miR-350 par rapport à un sujet sain indiquant une prédisposition à l'épilepsie, et ladite épilepsie étant une épilepsie post-traumatique (EPT).

2. Procédé de la revendication 1, dans lequel le miARN miR-340-3p présente :
i) entre 70 % et 80 % d'identité de séquence avec SEQ ID N° : 2,
ii) entre 80 % et 90 % d'identité de séquence avec SEQ ID N° : 2 ; ou
iii) entre 90 % et 100 % d'identité de séquence avec SEQ ID N° : 2.

3. Procédé de la revendication 1, dans lequel le miARN miR-484 présente
i) entre 70 % et 80 % d'identité de séquence avec SEQ ID N° : 5,
ii) entre 80 % et 90 % d'identité de séquence avec SEQ ID N° : 5 ; ou
iii) entre 90 % et 100 % d'identité de séquence avec SEQ ID N° : 5.

4. Procédé de la revendication 1, dans lequel le miARN miR-350 présente
i) entre 70 % et 80 % d'identité de séquence avec SEQ ID N° : 3 ou 4,
ii) entre 80 % et 90 % d'identité de séquence avec SEQ ID N° : 3 ou 4 ; ou
iii) entre 90 % et 100 % d'identité de séquence avec SEQ ID N° : 3 ou 4.

5. Procédé de l'une quelconque des revendications 1 à 4, comprenant en outre la détection d'un biomarqueur comprenant SEQ ID N° : 6 (miR-151a-3p).

6. Procédé de l'une quelconque des revendications 1 à 3, ledit individu étant un humain ou un rongeur.

7. Procédé de l'une quelconque des revendications 1 à 3, ledit individu ayant subi un traumatisme crânien et/ou une ou plusieurs commotions cérébrales.

8. Utilisation d'un kit destiné à être utilisé pour diagnostiquer la prédisposition à l'épilepsie post-traumatique (EPT) dans un échantillon de sang, le kit comprenant des composés pour la détection d'au moins un biomarqueur ARNm choisi parmi miR-340-3p (SEQ ID N° : 2), miR-484 (SEQ ID N° : 5) et/ou miR-350 (SEQ ID N° : 3 ou 4), ou une séquence présentant une identité de séquence de 80 % à 90 % avec l'une quelconque des SEQ ID N° : 2 à 5, et un échantillon témoin provenant d'un sujet sain, ledit échantillon étant un échantillon de sang et ladite détection d'une régulation à la hausse du miARN miR-340-3p, miR-484 et/ou miR-350 par rapport à un sujet sain indiquant une prédisposition à l'EPT.

9. Utilisation selon la revendication 8, dans laquelle le kit comprend des composés pour la détection de biomarqueurs constitués essentiellement de deux, trois ou des quatre biomarqueurs suivants présentant une identité de séquence de 80 % à 90 % avec SEQ ID N° : 2, SEQ ID N° : 3, SEQ ID N° : 4, SEQ ID N° : 5.

10. Utilisation selon la revendication 8 ou la revendication 9, dans laquelle le kit comprend en outre des composés pour la détection de miR-151a-3p (SEQ ID N° : 6), ou une séquence présentant une identité de séquence de 70 % à 80 % avec SEQ ID N° : 6.

11. Utilisation selon la revendication 10, dans laquelle le kit est constitué essentiellement de composés pour la détection de deux, trois ou quatre des biomarqueurs suivants : miR-340-3p, miR-484, miR-350 et/ou miR-151-3p.
